# EUROPEAN PATENT APPLICATION

(11) **EP 1 880 688 A1**
(43) Date of publication of application: **23.01.2008**
(21) Application number: 07110592.8
(22) Date of filing: 19.06.2007
(51) Int. Cl.: A61B 19/00, A61F 9/007, A61B 17/00

(54) **System and method for facilitating setup of surgical instrumentation and consumables associated therewith**

(30) Priority: 29.06.2006 US 477210
(71) Applicant: Alcon, Inc., 6331 Hünenberg (CH)
(72) Inventor: Ekvall, Johan, Laguna Beach, CA 92651 (US); Todd, Kirk, Yorba Linda, CA 92887 (US); Essex, Paul, Rancho Santa Margarita, CA 92688 (US); Gelland, Torsten, Irvine, CA 92612 (US)
(74) Representative: Hanna, Peter William Derek

(57) **Abstract**

The present invention provides a system and method useful in setting up a complex ophthalmic surgical instrumentation and consumables associated therewith. One embodiment is directed to a surgical system (500) capable of displaying a video (608) showing setup instructions corresponding to a consumable or a consumable pack. Each pack may contain one or more consumables. Once a consumable is scanned, selected, or otherwise identified, the surgical system may operate to query a database (604) to obtain Directions For Use (DFU) and other data associated with the consumable, update pack settings (605) accordingly, and provide setup instructions in various modes, including the Wizard (607). The Wizard provides setup instructions (e.g., visual, audio, text, etc.) one step at a time and displays the next step in a pre-defined setup sequence only when the preceding step has been correctly and successfully completed (610).

## Description

### FIELD OF THE INVENTION

The present invention relates generally to surgical instruments and procedures for setting up the same. More particularly, embodiments of the present invention relate to a system and method that can facilitate setting up surgical instruments and consumables associated therewith, particularly useful in setting up an ophthalmic surgical console.

### BACKGROUND OF THE INVENTION

The human eye can suffer a number of maladies causing mild deterioration to complete loss of vision. While contact lenses and eyeglasses can compensate for some ailments, ophthalmic surgery is required for others. Generally, ophthalmic surgery is classified into posterior segment procedures, such as vitreoretinal surgery, and anterior segment procedures, such as cataract surgery. More recently, combined anterior and posterior segment procedures have been developed.

The surgical instrumentation used for ophthalmic surgery can be specialized for anterior segment procedures or posterior segment procedures or support both. In any case, the surgical instrumentation often requires the use of associated consumables such as surgical cassettes, fluid bags, tubing, hand piece tips and so on. In some cases, a surgical console may house some or all of the associated surgical instrumentation and consumables and may provide a centralized system for monitoring and/or controlling the same.

The setup of an ophthalmic surgical console can be quite complex as setting up a surgical instrumentation generally involves various electrical cables and pneumatic/fluidic tubing, etc. The complexity of the setup can impact the speed and therefore productivity of the surgical staff. The aforementioned consumables often can cause further delay and perhaps confusion. Currently, "Directions for Use" (DFUs) for consumables are typically printed on a piece of paper or on the packaging material. The consumables may be taken out of the package for various reasons (e.g., use, storage, inventory, etc.). More often than not, the non-sterile packaging materials, along with the DFUs, are then discarded. In cases where the DFUs are printed on paper, they are likely to be misplaced. As a result, the DFUs for the consumables may be lost or otherwise not available at the time the surgical instrument is to be setup. Therefore, there is a need for a system and method that can facilitate the surgical staff in setting up surgical instrumentations and associated consumables in a more efficient, faster, and accurate way. Embodiments of the invention can address this need and more.

### SUMMARY OF THE INVENTION

The present invention provides a system, computer program and method for facilitating surgical staff to set up surgical instruments and consumables associated therewith, in accordance with claims which follow. Embodiments of the invention disclosed herein can provide video and other multimedia content to a user to aid the user in setting up surgical equipment, for example, in preparation for an operation. In particular, the video can provide a walkthrough of preparing surgical cassettes, hand pieces and other consumables used in ophthalmic surgery. Various embodiments of the present invention can allow a user, such as a nurse, to watch the entire setup video from beginning to end, watch each step of the setup in the video and complete that step before moving on to the next step, or watch only selected steps of the video. This can allow the user to tailor the tutorial to his or her level of experience.

One embodiment of the present invention can include receiving, at a surgical system, an identifier for one or more consumables, automatically obtaining consumable information associated with a first consumable from the one or more consumables from a database and displaying content comprising at least a video showing one or more steps in setting up said first consumable based on said consumable information.

Another embodiment of the invention is directed to a surgical system capable of displaying a video clip or other suitable media file showing setup instructions. The video clip corresponds to a consumable or consumable pack which can be scanned or selected by the user or otherwise recognized by the surgical system. The video clip showing the setup instructions may be displayed in one or more ways (e.g., with or without audio, text, graphics, etc.). The surgical system, according to one embodiment can include a display, a recognition device, and a controller operatively coupled to said display and said recognition device, wherein said controller comprises a processor and a computer-readable medium carrying program instructions executable by said processor. The program instructions can comprise, code for receiving an identification of a first consumable pack which contains a set of consumables, code for automatically obtaining consumable information associated with said set of consumables, code for updating settings of said surgical system based on said consumable information and code for displaying on said display content comprising at least a video showing a set of steps in setting up the set of consumables.

In embodiments of the invention, the control of the video clip or other suitable media file can be tailored to suit the various needs, knowledge, and/or skill levels of users. In one embodiment, the surgical system can provide at least three modes: Advanced, Video, and Wizard. Any one of the modes can be configured as the default mode. In the first mode, a user can skip the playback of the audio/video clip entirely and simply jump to or select a desired point or topic related to a particular instrument or consumable. In the second mode, a user can choose to play the entire clip without interruption. In the third mode, step-by-step instructions are displayed and, optionally, a user may be required to acknowledge at the completion of each step in order to move forward to the next step in a pre-defined setup sequence.

In one embodiment, the surgical system, through computer-executable program instructions embodied on a computer-readable medium residing therein, may operate to move to the next step in the setup sequence upon detecting a correct completion of the preceding step (e.g., the correct installation of a surgical cassette). The interactivity between the user and the surgical system may continue until all steps necessary for setting up a surgical instrument, a selected or scanned consumable pack, or the surgical console have been completed.

One advantage provided by embodiments of the present invention relates to a surgical system's ability of facilitating a user to setup complex ophthalmic surgical instrumentation and consumables associated therewith.

Another advantage provided by embodiments of the present invention relates to a surgical system's flexibility of facilitating diverse users with various levels of skills in setting up complex ophthalmic surgical instrumentation and consumables associated therewith.

Moreover, embodiments of the invention can help to prevent errors due to misconnection of consumables, prevent improper setup of surgical instrumentation, eliminate problems associated with the loss of paper DFUs, streamline the surgical instrumentation setup process, and increase the speed of setting up a surgical console.

Other advantages of the present invention will become more apparent to one skilled in the art upon reading and understanding the detailed description of the preferred embodiments described herein with reference to the following drawings.

### BRIEF DESCRIPTION OF THE DRAWINGS

A more complete understanding of the present invention and the advantages thereof may be acquired by referring to the following description, taken in conjunction with the accompanying drawings in which like reference numbers indicate like features and wherein:
FIGURE 1 is a diagrammatic representation of one embodiment of a surgical console;
FIGURE 2 is a diagrammatic representation of one embodiment of a cassette receiver;
FIGURE 3 is a diagrammatic representation of one embodiment of a surgical cassette;
FIGURE 4 is a diagrammatic representation illustrating a cross-sectional view of one embodiment of a cassette in a cassette receiver;
FIGURE 5 is a diagrammatic representation of one embodiment of a surgical system that can be implemented as a surgical console;
FIGURE 6 is a flow chart illustrating method steps operable to facilitate a user in setting up surgical instrumentation and consumables associated therewith;
FIGURES 7-9 are screenshots of one embodiment of a user interface implementing the surgical system of FIGURE 5 and the method of FIGURE 6; and
FIGURE 10 is a screenshot of a user interface implementing the surgical system of FIGURE 5, according to another embodiment of the invention.

### DETAILED DESCRIPTION OF THE INVENTION

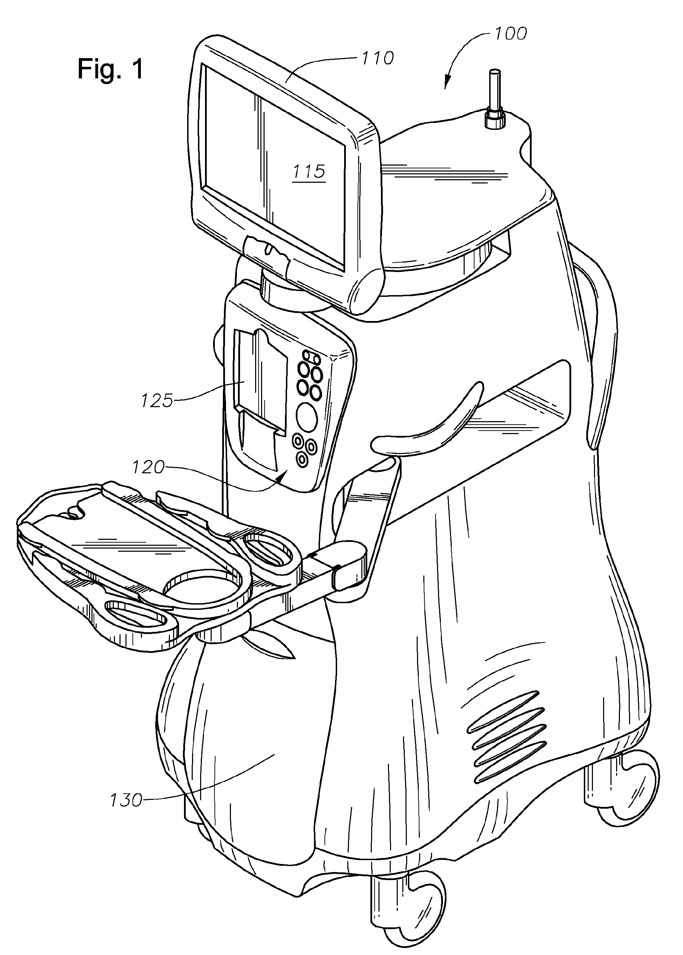

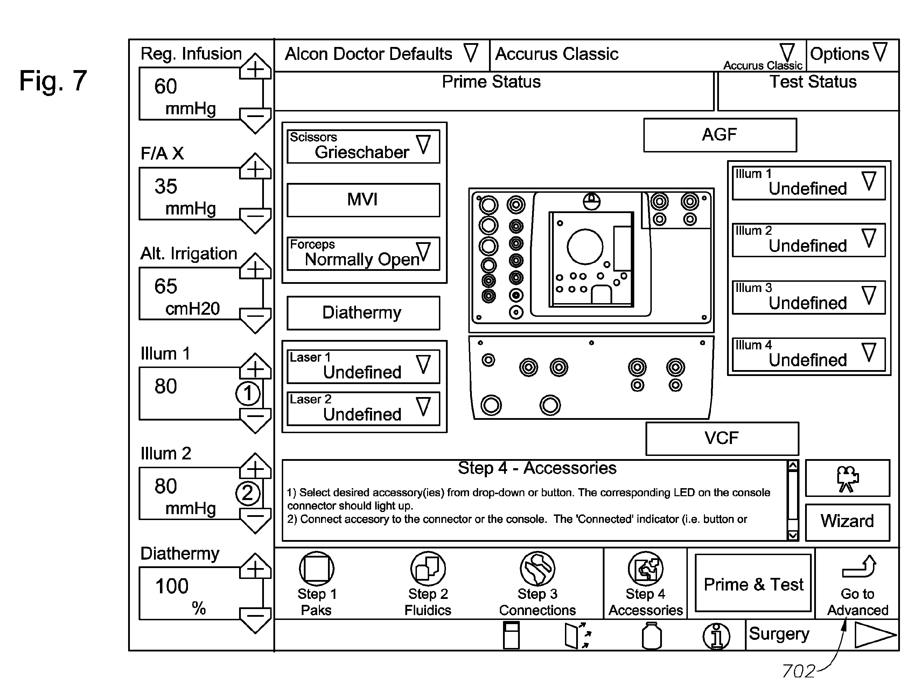

Embodiments of the invention disclosed herein provide a system and method for facilitating setup of consumables associated with surgical instrumentation. FIGURES 1-4 illustrate one example of a surgical system and a related consumable, in this case a cassette. These drawings exemplify a surgical environment in which embodiments of the present invention may be implemented. It can be seen from these FIGRUES that a surgical console acts in cooperation with a number of consumables that require setup before a surgical procedure takes place. Embodiments of the present invention provide a system and method for facilitating setup of the consumables through video and multimedia content displayed on a surgical console. As described further below, the console can recognize the consumables to be used through a variety of mechanisms and play the appropriate content for the user to aid the user in setting up the consumables. FIGURES 7-10 provide example embodiments of displaying multimedia content to the user to make setup of surgical equipment more efficient and less error prone. Through the use of videos and other multimedia content, the present invention provides a mechanism that facilitates setup and eliminates, or at least substantially reduces, the shortcomings of previous methods for providing instructions.

One embodiment of the invention is directed to a surgical system capable of displaying a video clip or other suitable media file showing setup instructions. The video clip corresponds to a consumable or consumable pack which can be scanned or selected by the user or otherwise recognized by the surgical system. The invention and various features and advantageous details thereof are explained more fully with reference to the exemplary, and therefore non-limiting, embodiments illustrated in the accompanying drawings and detailed in the following description.

Descriptions of known programming techniques, computer software, hardware, operating platforms and protocols may be omitted so as not to unnecessarily obscure the invention in detail. It should be understood, however, that the detailed description and the specific examples, while indicating the preferred embodiments of the invention, are given by way of illustration only and not by way of limitation. Various substitutions, modifications, additions and/or rearrangements within the spirit and/or scope of the underlying inventive concept will become apparent to those skilled in the art from this disclosure.

FIGURE 1 is a diagrammatic representation of one embodiment of an ophthalmic surgical console 100. Surgical console 100 can include a swivel monitor 110 that has touch screen 115. Swivel monitor 110 can be positioned in a variety of orientations for whomever needs to see touch screen 115. Swivel monitor 110 can swing from side to side, as well as rotate and tilt. Touch screen 115 provides a graphical user interface ("GUI") that allows a user to interact with console 100.

Surgical console 100 also includes a connection panel 120 used to connect various tools and consumables to surgical console 100. Connection panel 120 can include, for example, a coagulation connector, balanced salt solution receiver, connectors for various hand pieces and a fluid management system ("FMS") or cassette receiver 125. Surgical console 100 can also include a variety of user friendly features, such as a foot pedal control (e.g., stored behind panel 130) and other features.

In operation, a cassette (not shown) can be placed in cassette receiver 125. Clamps in surgical console 100 clamp the cassette in place to minimize movement of the cassette during use. The clamps can clamp the top and bottom of the cassette, the sides of the cassette or otherwise clamp the cassette.

Surgical console 100 is provided by way of example and embodiments of the present invention can be implemented with a variety of surgical systems. Example surgical systems in which cassettes according to various embodiments of the present invention can be used include, for example, the Series 2000® Legacy® cataract surgical system, the Accurus® 400VS surgical system, and the Infiniti^{™} Vision System surgical system, all available from Alcon Laboratories Inc. of Fort Worth, Texas. Additionally, embodiments of the present invention can be used with a variety of surgical cassettes, examples of which are described in U.S. Pub. Nos. 2005/0186098 (Application No. 11/114,289 to Davis et al.), 2004/0253129 (Application No. 10/891,642 to Sorensen et al.), 2005/0065462 (Application No. 10/979,433 to Nazarifar et al.), 2003/0225363 (Application No. 10/156,175 to Gordon et al.), 2001/0016711 (Application No. 09/846,724 to Sorensen et al.) and United States Patent Nos. 6,293,926 to Sorensen et al., 4,493,695 to Cook, 4,627,833 to Cook, 4,395,258 to Wang et al., 4,713,051 to Steppe, et al., 4,798,850 to DeMeo, et al., 4,758,238 to Sundblom et al., 4,790,816 to Sundblom et al., 6,267,956 to Beuchat, 6,364,342 to Beuchat, 6,036,458 to Cole et al., and 6,059,544 to Jung et al. Embodiments of the present invention can be implemented for other suitable surgical systems and cassettes as would be understood by one of ordinary skill in the art.

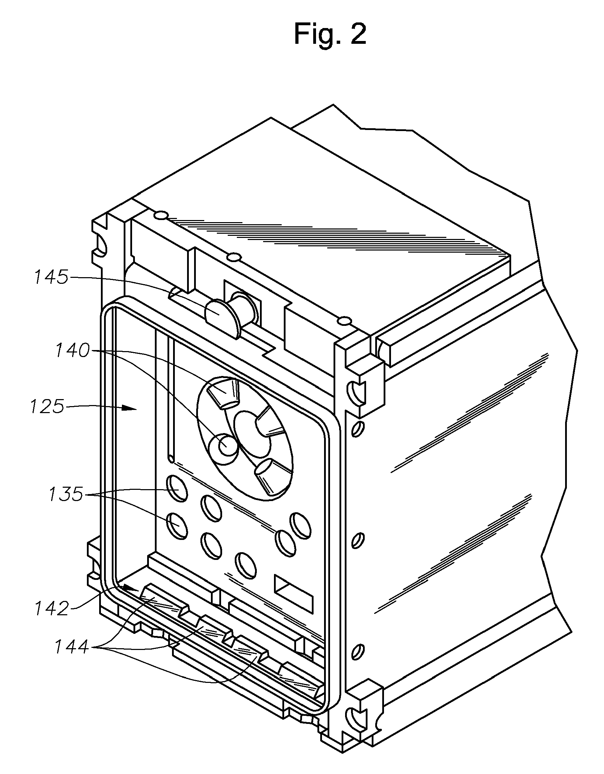

FIGURE 2 is a diagrammatic representation of one embodiment of cassette receiver 125 without a cassette. Cassette receiver 125 can have various input and output ports (indicated generally at 135) to receive fluids (i.e., liquids and gasses) from the surgical cassette. Cassette receiver 125 can further include an opening to allow peristaltic pump rollers 140 to contact the surgical cassette during operation. One embodiment of a peristaltic pump and complimentary cassette is described in United States Patent No. 6,293,926 to Sorensen.

The surgical cassette, in the embodiment of FIGURE 2, is held in place by a clamp having a bottom rail 142 and a top rail (not shown). Each rail can have clamping fingers (e.g., clamp finger 144) that contact the cassette in corresponding clamping zones. One embodiment of a surgical cassette clamp is described in United States Patent Application Publication No. 2003/0202894 (Serial No. 10/132,797). A release button 145 is pressed to initiate release of the cassette from the clamp. Depending on the surgical console 100, the cassette release process can include several steps, including venting of pressure or fluids, disengaging the clamps or other steps. The configuration of FIGURE 2 is provided by way of example. The form factor of cassette receiver 125, placement and number of input/output ports and other features of cassette receiver 125 can depend on the surgical console 100, on the surgical procedure being performed or on other factors.

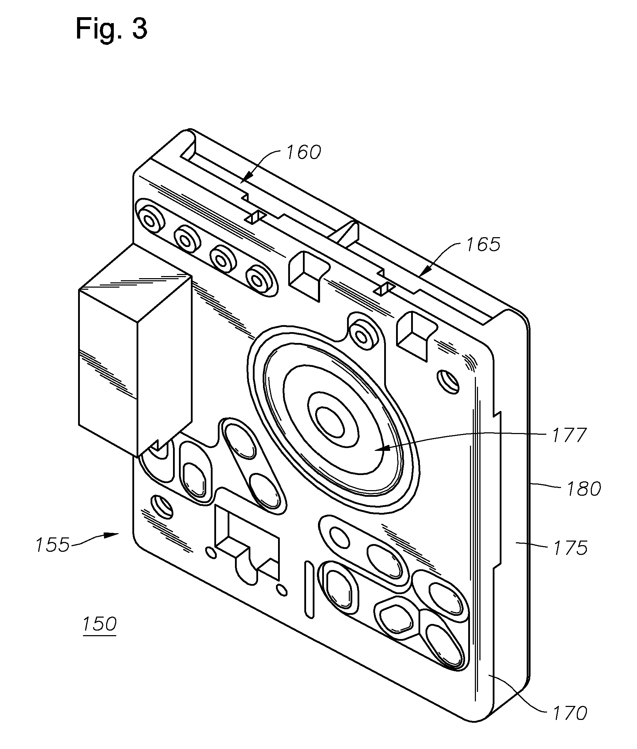

FIGURE 3 is a diagrammatic representation of one embodiment of a surgical cassette 150. Cassette 150 can provide a closed system fluidic device that can be discarded following a surgical procedure. Cassette 150 can include a cassette body 155 and clamp receiving portions (e.g., indicated generally at clamping zones 160 and 165) projecting from the cassette body 155. In the embodiment shown, cassette 150 is formed from three primary sections: an inner or surgical console interface section 170 that faces the surgical console when cassette 150 is inserted into surgical console 100, a middle section 175 and a back plate 180. The various sections of cassette 150 can be coupled together via a press fit, interlocking tabs, chemical bonding, thermal bonding, mechanical fasteners or other attachment mechanism known in the art.

Surgical console interface section 170 can provide an interface for fluid flow channels (e.g., flow channel 177 for the peristaltic pump provided by an elastomeric pump membrane), valves (e.g., irrigation/aspiration valves), pressure sensors and other features to manage fluid flow. Cassette 150 can also attach to a fluid bag (not shown) to collect fluids during a procedure.

In operation, cassette 150 is held in place in cassette receiver 125 by clamp rails that contact cassette 150 in the clamping zones. For example, the upper clamp rail will contact cassette 150 in clamping zone 160 and clamping zone 165 while the bottom clamp rail (e.g., bottom clamp rail 142) will contact cassette 150 at similar bottom clamping zones.

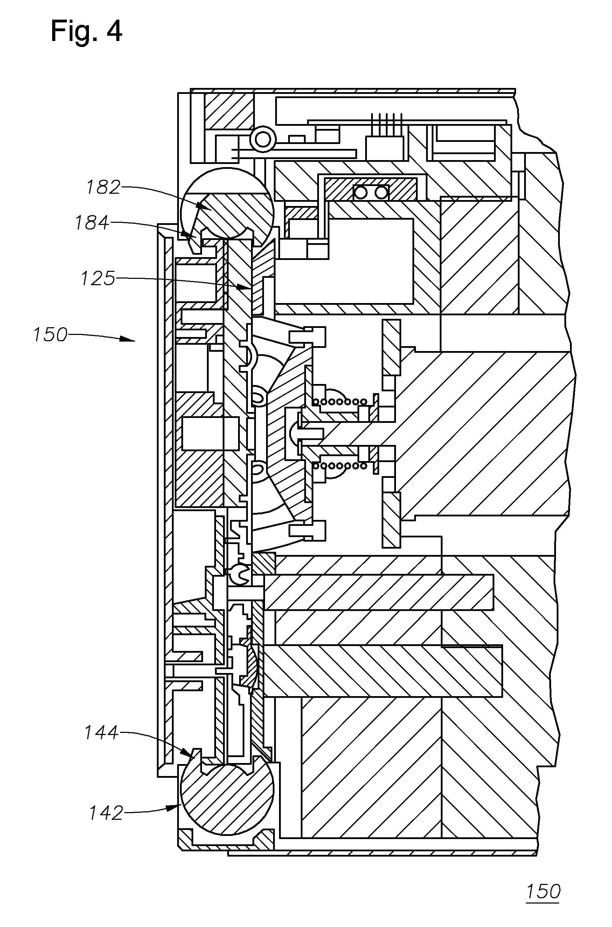

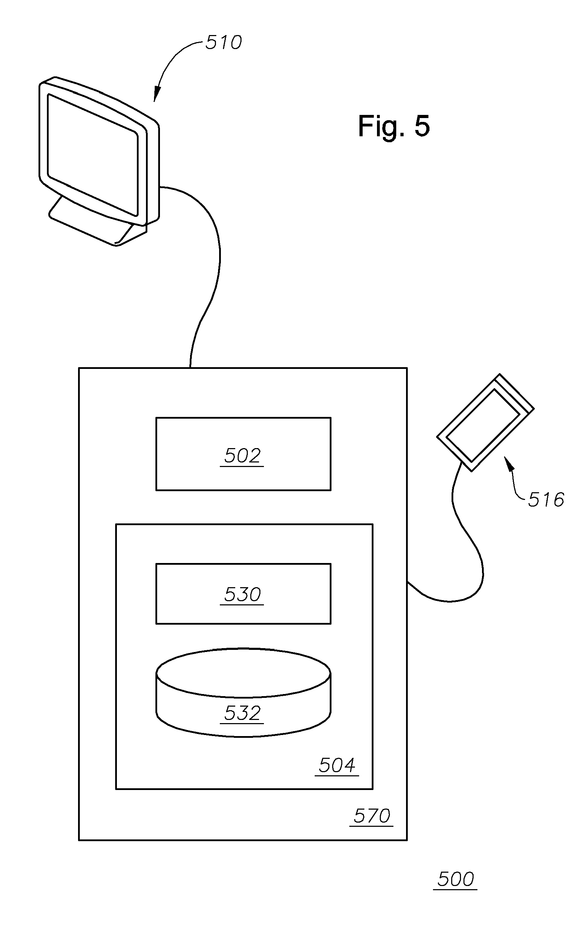

FIGURE 4 is a cross-section of one embodiment of cassette 150 inserted into cassette receiver 125. Cassette 150 is held in place by a clamp. In the embodiment of FIGURE 5, the clamp includes lower clamp rail 142 and upper clamp rail 182, though in other embodiments the clamp can contact cassette 150 in other areas. When cassette 150 is initially inserted, clamp rails 142/182 rotate so that clamping fingers (e.g., clamp finger 144 and clamp finger 184) contact cassette 150 in the clamping zones. Rotation can be imparted to the clamp rails 142/182 from the force of insertion, by an air cylinder, by a motor or any combination of these. To release the cassette, the clamp rails 142/182 rotate in the opposite direction. When inserted, surgical console interface section 155 can contact surgical console 100 such that, for example, peristaltic pump rollers 140 can squeeze flow channel 177.

Thus, it can be seen from the previous FIGURES that setting up surgical instrumentation can take several steps, including, but not limited to, connecting hand pieces through various connection ports and inserting cassettes. Depending on the consumables used, the setup steps may vary. As described below, embodiments of the present invention can facilitate and streamline the setup process of surgical instrumentation through displaying video clip(s) or other suitable media showing appropriate setup instructions that correspond to the consumable(s) being used in connection with the surgical instrumentation. Optionally, content as well as the manner in which setup instructions are provided can depend on the sophistication of the user.

FIGURE 5 is a diagrammatic representation of one embodiment of a surgical system 500 that can be implemented in or as a surgical console (e.g., surgical console 100 of FIGURE 1) to facilitate setup of surgical instrumentation through the use of videos and other multimedia content. In one embodiment, surgical system 500 comprises a controller or functional block 570, a display 510, and a consumable pack recognition device 516. In one embodiment, functional block 570 comprises a processor 502 and a computer-readable medium 504 carrying program instructions 530, which are executable by processor 502. In one embodiment, computer-readable medium 504 may implement a data structure 532. In one embodiment, data structure 532 can be a searchable database storing information pertaining to a plurality of consumable packs. Other suitable data structures (e.g., files) can also be used.

In practice, each consumable pack may contain one or more consumables or consumable items. Embodiments of the present invention can recognize the consumable pack (or individual consumable items) and play appropriate content for setting up the consumables. In one embodiment, consumable pack recognition device 516 can be a barcode scanner, RFID recognition device or other device. In one embodiment, database 532 can be programmed to be searchable via a scanned barcode, RFID, key or other search parameter. Other suitable search techniques (e.g., such as searching by radio frequency identifications (RFID)) can be used as would be recognized by those skilled in the art. In one embodiment, functional block 570 is connected to display 510. In one embodiment, similar to swivel monitor 110 of FIGURE 1, display 510 implements a touch screen for providing an interactive GUI that allows a user to interact with surgical system 500 and hence console 100, which embodies surgical system 500.

Program 530 and database 532 may be stored at a number of different locations and executed in a distributed manner. Database 532 may reside on the same computer-readable medium as program 530 or it may reside on a separate computer-readable medium internal or external to device 570 or surgical system 500. Moreover, either or both program 530 and database 532 could be part of a larger program, can comprise separate programs operable to communicate data to each other, or can be implemented according to any suitable programming architecture and language.

As to the manner in which a consumable pack is recognized by surgical system 500, any commercially available or known product identification or product recognition methodology and apparatus may be adapted or otherwise suitably implemented in embodiments of the invention, as long as consumable pack recognition device 516 can identify consumable packs for surgical system 500. For example, smart tags, RFID tags or labels and corresponding sensors may be utilized in place of or in addition to barcodes and barcode scanners. Additionally, the user can input an indication of which consumables are to be used (e.g., by entering an item number or SKU via the SUI). So long as display 510 can allow a user to interact with surgical system 500, the formats, styles, sizes, colors, features, and configuration of the GUI and the touch screen of display 510 may vary from one implementation to another.

When a consumable pack has been recognized, whether through barcode, RFID tag, smart tag, user input or other identification of the consumable pack, program 530 can access database 532 to determine which media files to play for the associated consumables. The order in which particular media files are played can depend on the consumables in the consumable pack. In operation, the media files are played to provide a tutorial to a user on the setup of the consumables. Through the use of media content, embodiments of the present invention increase the efficiency of the setup procedure. Moreover, unlike paper DFUs, the setup instructions are not easily lost or misplaced as they are stored as computer data, preferably on the surgical console that is being setup.

As one skilled in the art can appreciate, embodiments of the invention disclosed herein can be modified or otherwise implemented in many different ways without departing from the scope of the invention. For example, computer-executable instructions stored on a computer-readable medium may reside on a variety of computing devices and/or in various computing environments. Program 530 may be executable to receive data from and store data to database 532 over a network in which surgical system 500 resides.

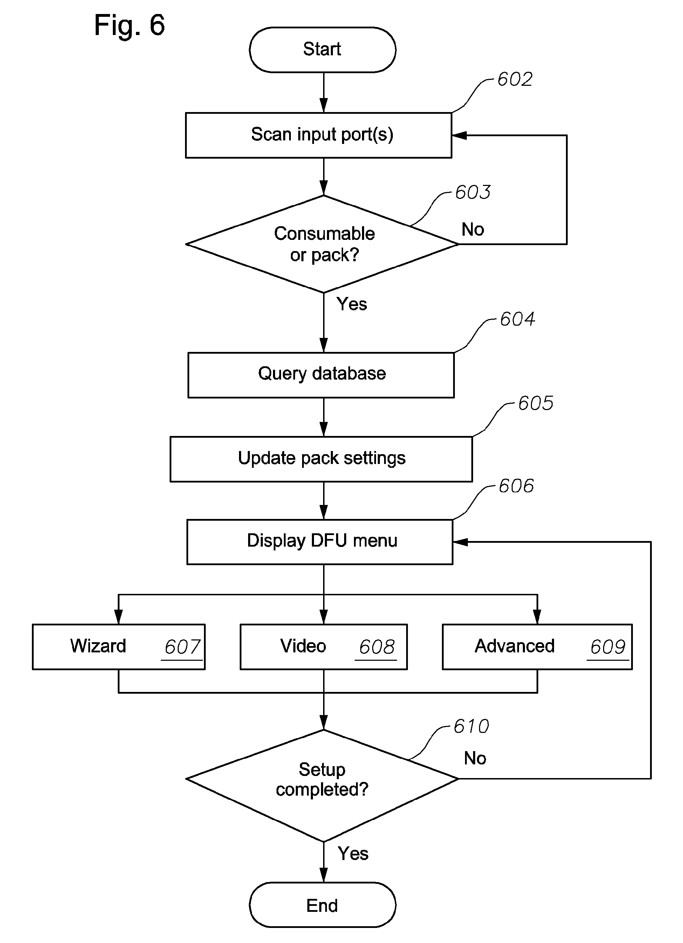

FIGURE 6 is a flow chart illustrating method steps operable to facilitate a user of surgical system 500 to set up surgical instrumentation and consumables associated therewith. The method of FIGURE 6 can be implemented as a set of computer executable instructions stored on a computer readable medium at surgical system 500. In one embodiment, a method of setting up surgical instrumentation may comprise identifying (e.g., via scanning or selecting) a first consumable or a first consumable pack. The first consumable pack may comprise one or more consumable items. In one embodiment, surgical system 500 may be configured to monitor or scan (Step 602) an input port of a surgical console (e.g., surgical console 100 of FIGURE 1) to automatically detect and receive (Step 603) a signal or signals (e.g., an identifier or identification of a consumable or consumable pack) from a recognition device (e.g., barcode scanner 516, RFID tag recognition device or other device known or developed in the art). In another embodiment, surgical system 500 may be programmed to allow a user to manually enter a code for a consumable or a consumable pack. In yet another embodiment, surgical system 500 may be programmed to allow a user to select a consumable or a consumable pack from a menu of consumables and/or consumable packs. Other ways to enable a user to scan or select the first consumable or first consumable pack are also possible.

In one embodiment, once the identity of the first consumable or the first consumable pack is known, the method may further comprise querying a database and obtaining from the database information associated with the consumable or with the first consumable pack (Step 604). In one embodiment, the querying can be accomplished by looking up consumable information stored in the database. Information obtained at Step 604 may be utilized to update pack-dependent settings at Step 605 (e.g., probe types, handpieces, video clips to be played). Additionally, information obtained at Step 604 can include Directions for Use (DFU) associated with one or more consumable items (e.g., from the consumable pack).

The method may further comprise displaying a menu on display 510 with information obtained at Step 604 and may include running an application on surgical system 500 to provide a step-by-step guide in setting up the first consumable or the one or more consumables in the first consumable pack (Step 607). The wizard application can utilize data obtained from database 532 to provide instructions on how to setup consumables. Surgical system 500 may operate to detect the completion of each step and play audio/video, image(s), text or a combination thereof showing the next step in the setup sequence. In one embodiment, the method may include a step of automatically playing on display 510 a video clip and other media associated with identified consumables (Step 608). The method may further include a step of enabling to control playing of the video or other media. If all DFU steps are completed, the method may end (Step 610) or proceed to allow the next consumable or consumable pack to be scanned, selected, or otherwise identified. The steps of FIGURE 6 can be repeated as needed or desired.

As discussed above, embodiments of the present invention facilitate setup of surgical instrumentation by providing media content (e.g., video, audio, multimedia content) associated with consumable items. The media content can provide a tutorial on the setup of the consumable items. FIGURES 7-9 are screenshots of a user interface (UI) (e.g., of surgical system 500) for providing media content to a user to facilitate setup of consumables. As illustrated in FIGURE 7, the UI of surgical system 500 may provide, through computer-executable program instructions embodied on a computer-readable medium residing on surgical system 500, a plurality of functional selections (e.g., drop-down menus, buttons, etc.) to suit users with different needs and levels of skills and knowledge in instrumentation setup procedures. In general, the UI provides videos and other content to the user to facilitate setup of consumables. As discussed above, the content displayed can depend on the consumable or consumables packs identified for use with a surgical procedure. In one embodiment, surgical system 500 may comprise at least three modes: Advanced, Video, and Wizard.

In the Advanced mode (e.g., by selecting the "Go To Advanced" button as depicted in FIGURE 7 at 702), a user does not have to watch the entire audio/video clip (the Video mode) or follow the step-by-step instruction (the Wizard mode). Instead, in the Advanced mode, a more experience user can quickly jump to or select a desired point or topic related to a particular instrument or consumable. The content for that selected topic can be played for the user. Thus, for example, a user who only wishes to see video associated with setting up the surgical cassette can skip to that content.

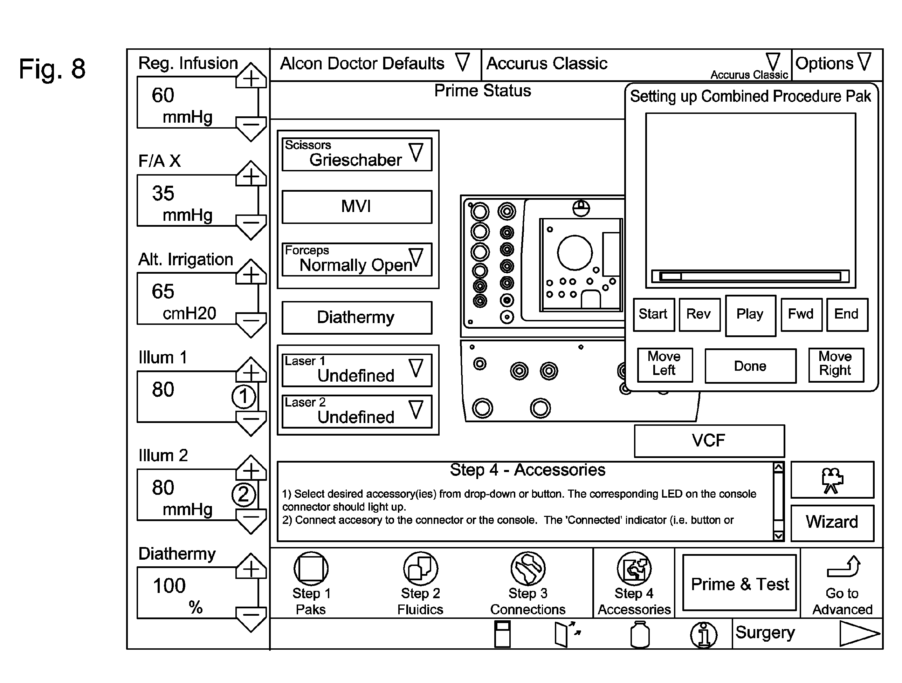

In the Video mode, a user who just wants a quick review of the setup steps can choose to play the entire video without interruption. As illustrated in FIGURE 8, the Video mode allows a user to view and review a media file or audio/video clip associated with a consumable pack (e.g., "Combined Procedure Pak" in FIGURE 8) in various ways (e.g., "Start", "Rev", "Play", "Fwd", "End", "Move Left", "Move Right", "Done", etc.).

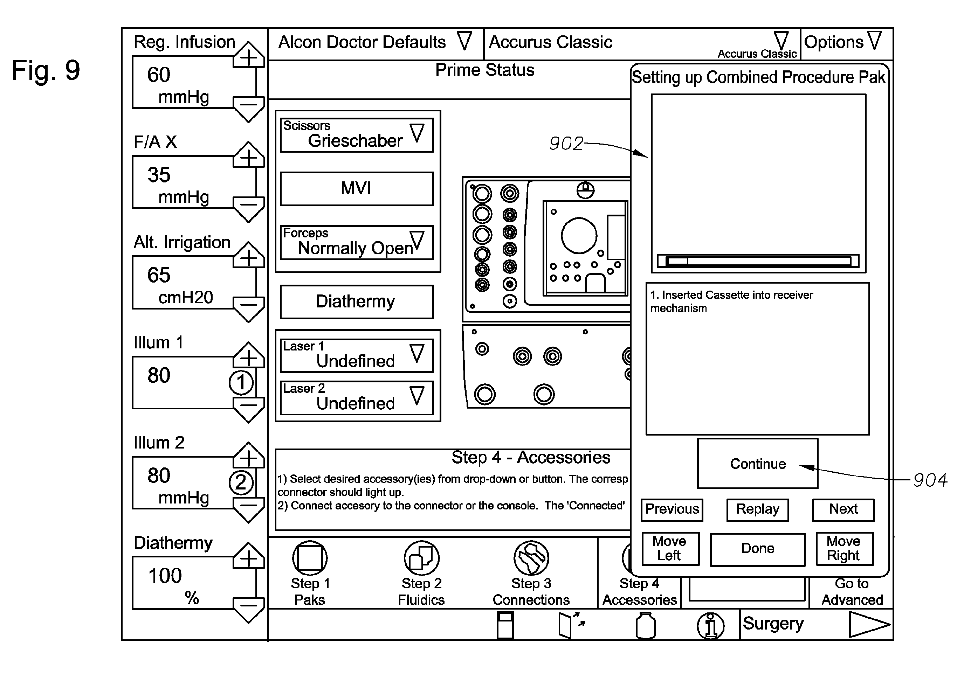

In the Wizard mode, a user is given setup instructions one step at a time (e.g., "1. Insert Cassette into receiver mechanism."), as illustrated in FIGURE 9 at area 902. The wizard can provide step-by-step instructions with the same or similar content shown in the Video mode. The wizard can be configured so that a user would need to acknowledge at the completion of each step in order to move forward (e.g., by pressing the "Continue" button 904). The acknowledgement can help to prevent an inexperienced user from making mistakes. After each step is completed, the next step in the setup sequence is displayed and the user again follows the setup instruction displayed. The interactivity between the user and the UI of surgical system 500 may repeat and/or continue until all steps necessary for setting up surgical console 100 or consumable operation are completed. In another embodiment, the surgical console 100 can detect completion of a step. Once the user has completed a step, the UI can display content for the next step.

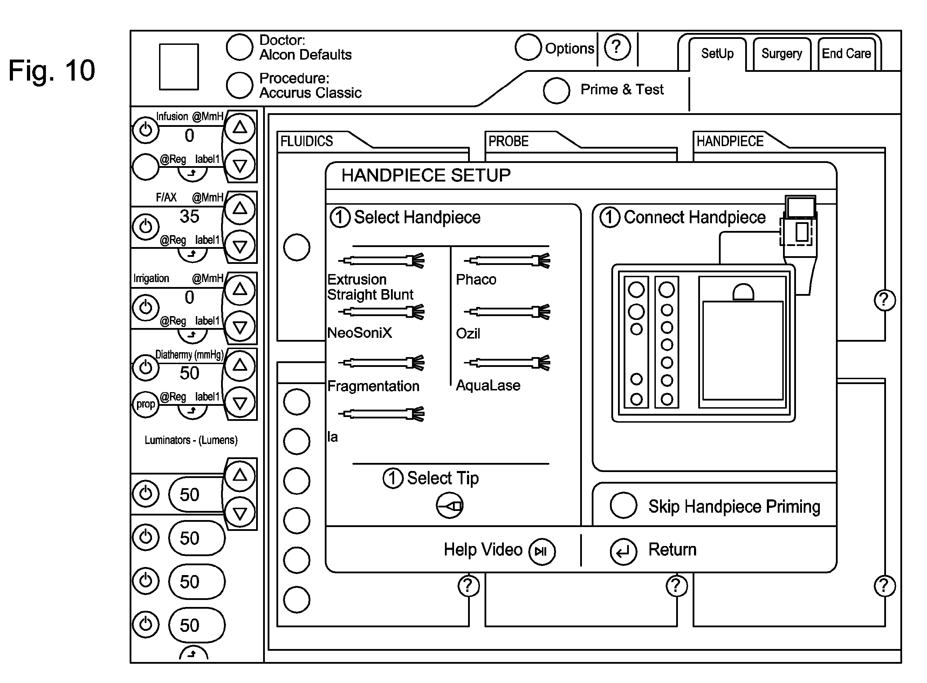

As one skilled in the art can appreciate, the UI of surgical system 500 may vary from one implementation to another. As an example, FIGURE 10 is a screenshot of a user interface implementing surgical system 500, according to another embodiment of the invention. In this case, a user can select a "Help Video" button at the bottom of a popup window "Handpiece Setup" on display 510. Upon the selection of the "Help Video" button, a media file or audio/video clip may be automatically presented to the user in a manner similar to the Video mode described above. Alternatively, the user may be presented with several options such as "Play Video", "Run Wizard", etc. Other implementations are also possible.

Embodiments of the invention described above can be a user-friendly replacement or addition to the DFUs located on the consumable pack. Moreover, embodiments of the invention can help to prevent errors due to misconnection of consumables, prevent improper setup of a surgical console, eliminate problems associated with the loss of paper DFUs, streamline the setup process, and increase the speed of setting up the surgical instrument. The functionality of the underlying surgical system extends to cover a wide range of skill levels and knowledge of users (e.g., the surgical staff working in an operating room). Embodiments of the invention can be particularly useful in facilitating the surgical staff to setup complex surgical instrumentation and consumables associated therewith correctly and efficiently.

## Claims

1. A method for facilitating setup of consumables associated with surgical instrumentation, comprising:
receiving, at a surgical system (500), an identifier for one or more consumables;
automatically obtaining consumable information associated with a first consumable from the one or more consumables from a database (532); and
displaying content (606) comprising at least a video (608) showing one or more steps in setting up said first consumable based on said consumable information, wherein the content is displayed in a display (510) of the surgical system.

2. The method of Claim 1, wherein displaying content comprising at least a video further comprises:
displaying content for a first step;
determining (610) that a user has completed the first step; and
displaying content (606) for a subsequent step.

3. The method according to claim 2, wherein said determining step (610) further comprises prompting a user to indicate completion of said first step.

4. The method according to claim 1, further comprising:
configuring said surgical system to allow a user to control playback (609) of said video (608).

5. The method according to claim 1, wherein said first consumable is a part of a consumable pack which includes one or more consumables.

6. The method according to claim 1, wherein said consumable information comprises Directions For Use corresponding to said first consumable.

7. The method according to claim 1, further comprising:
querying (604) said database (532) utilizing said identifier.

8. The method according to claim 1, wherein the identifier for the one or more consumables is a consumable pack identifier.

9. The method according to claim 1, further comprising:
updating settings (605) of said surgical system based on said consumable information.

10. A computer program (530) comprising instructions for implementing a method for facilitating setup of consumables associated with surgical instrumentation, wherein said program instructions comprise the steps of:
receiving (602,603), at a surgical system (500), an identification of a first consumable or a first consumable pack which contains said first consumable;
automatically obtaining consumable information (604) associated with said first consumable from a database (532); and
displaying content (606) comprising at least a video (608) showing one or more steps in setting up said first consumable based on said consumable information.

11. The computer program of Claim 10, further comprising the steps of:
detecting completion (610) a said first step; and
displaying content for a second step after the first step is completed.

12. The computer program of claim 11, further comprising the step of prompting a user to indicate completion of said first step.

13. The computer program of claim 10, wherein said displaying content comprises displaying (606) Directions For Use corresponding to said first consumable.

14. The computer program of claim 10, further comprising the step of enabling a user of said surgical system to select or skip (609) a media file or a portion thereof, wherein said media file corresponds to said Directions For Use.

15. The computer program of claim 10, further comprising the step of enabling a user to select, enter, or scan (602) said identification of said first consumable or said first consumable pack.

16. The computer program of claim 10, further comprising the step of querying (604) said database (532) utilizing said identification.

17. The computer program of claim 10, further comprising the step of updating settings (605) of said surgical system (500) based on said consumable information.

18. The computer program of any one of claims 10 to 17, when stored on a computer readable medium (504).

19. A surgical system (500) for facilitating setup of consumables associated with surgical instrumentation, comprising:
a display (510);
a recognition device (516); and
a controller (570) operatively coupled to said display and said recognition device, wherein said controller comprises a processor (502) and a computer-readable medium (504) carrying program instructions (530) executable by said processor, and wherein said program instructions comprise:
code for receiving (602,603) an identification of a first consumable pack which contains a set of consumables;
code for automatically obtaining consumable information (604) associated with said set of consumables from a set of consumable information (532) stored onboard the surgical system;
code for updating settings (605) of said surgical system based on said consumable information; and
code for displaying on said display content (606) comprising at least a video (608) showing a set of steps in setting up the set of consumables.

20. The surgical system of Claim 19, further comprising code to allow the user to play a video showing each of the set of steps without interruption (607).

21. The surgical system of Claim 19, further comprising code to allow the user to select (609) one or more of the set of steps for which the content will be displayed.

22. The surgical system of Claim 19, further comprising:
a) code for displaying content associated with a current step;
b) code for determining (610) that the user completed the current step; and
c) code for repeating steps a-b for each step in the set of steps.

23. The surgical system of claim 19, wherein said consumable information comprises Directions For Use corresponding to said first consumable.

24. The surgical system of claim 19, wherein said computer-executable program instructions further comprise code for enabling a user of said surgical system to select or skip (609) a media file or a portion thereof, wherein said media file corresponds to said Directions For Use.

25. The surgical system of claim 19, wherein said computer-executable program instructions further comprise code for enabling a user to select, enter, or scan (602) said identification.

26. The surgical system of claim 19, wherein said computer-executable program instructions further comprise code for querying (604) said set of consumable information using said identification.
